# EUROPEAN PATENT APPLICATION

(11) **EP 1 275 710 A1**
(43) Date of publication of application: **15.01.2003**
(21) Application number: 00908918.6
(22) Date of filing: 10.03.2000
(51) Int. Cl.: C12N 1/14, C12M 1/16

(54) **THE DEVICE FOR CULTIVATING CORDYCEPS SINENSIS AND METHOD FOR CULTIVATING IT AND THE USES THEREOF**

(71) Applicant: Sun, Shaosheng, Xixia City, Shandong Province 265303 (CN)
(72) Inventor: SUN, Shaosheng, Xixia City, Shandong Province 265303 (CN); SUN, Lijun, Xixia City, Shandong Province 265303 (CN); SUN, Liping, Xixia City, Shandong Province-265303 (CN); SUN, Lige, Xixia City, Shandong Province 265303 (CN)
(74) Representative: Rupp, Christian, Dipl.-Phys.
(86) International application number: CN0000050
(87) International publication number: WO01066692

(57) **Abstract**

This invention relates to the device and protocol for Cordyceps sinensis culture and the product uses thereof. The method comprises the following steps: collecting different species sources of Cordyceps sinensis within the same genus; producing good quality hybrid strains via hybridization culture; producing primordium fruitbodies via inoculation of the hybrid strains into the culture media using the tailor-made cultivation container; producing the fruitbodies of the present invention via placing the container into the cultivation incubators or chambers with optimized temperature, humidity, lighting and air conditions which can ensure the normal growth and development of the fungus; the resultant fruitbodies in this invention are suitable for applications in manufacturing of foods, tonics, cosmetics, and immunization products or medicines with the advantages of higher nutrition and remedial uses and lower production costs.

## Description

### Technical areas of the invention

This invention involves techniques for microorganism culture, particularly referred to as a device and method for Cordiceps sinensis Culture and the uses thereof

### Technical Backgrounds of the invention

It is well known that Cordiceps sinensis is a rare medicine with multiple remedial efficacies such as immunologic enhancement, nutritious supplements, cure or prevention of some diseases, cancer preventions or defenses, and anti-aging etc. due to its abundance of nucleotides and cordycepin. But it has hitherto not been possible to artificially cultivate this precious fungus ,as it is mainly grown in the wide environment and the resource is gradually decreasing as a result of extensive harvests. It was known that the mycelium had been successfully produced via fermentation engineering techniques in Jiangxi Guoyao Pharmacy Factory, but their method failed to gain the fruitbodies (the "grass" potion). Researchers in Jinin Silkworm Research Institute were successful in cultivation and patent protection of their fruitbodies using silkworm chrysalis as the hosts. However, the major weakness of their method is that their production activities were restricted by the propagating seasons and areas due to of the use of the silkworm as the host and that the yields were limited. Furthermore, the production costs were also higher.

### The technical schemes for the invention

The technical schemes contained in this invention are the fruit of several decades of repeated research and experimenting on the part of the authors.

The first major purpose of this invention was to provide a device for the cultivation of the Cordiceps sinensis by means of research on the physiological features and the behavior of this fungus; a device was designed to maintain its year-round growth and development by establishing a suitable ecological environment.

Another major purpose of this invention was to provide a culture method for Cordiceps sinensis. Through collections and hybridization cultures of different species sources of Cordyceps Sinensis within the same genus, we first gained the good-quality species of the fungus. Then the hybrid strains were inoculated into specially-designed culture containers containing the culture media so as to gain the primordium stromas. After obtaining the primordium fruitbodies, we placed them in incubators or chambers of this invention. The temperature, humidity, light and air were optimized to achieve suitable conditions for ensuring the growth and development of the fruitbodies. We then received the products which we were seeking.

The invention was achieved as follows:
Firstly, we needed to make utensils for our cultivations. The major modules of the utensils included incubator bodies and cultivation containers in these. The body of an incubator comprised a base and a main body. The main body comprised door(s) and at least one interior layer for holding the containers; inlets and outlets for gases were inserted and connected with ventilation facilities on the sides, while air conditioning units for controlling the temperature and humidity together with lighting facilities inside the body were added. The cultivation container was made up of a bottom and a lid comprising many holes with elastic plugs in them.
There were many apertures running through the interior layer(s), or reticulation structures were used. The main bodies of the incubators were transparent or opaque; the lids of the cultivation containers were of transparent structures while the bottoms were transparent or opaque.

In additional to the above invention, we also created a cultivation method for the Cordiceps sinensis as follows:
(1) Collections of fungus strains
Different species of wide Cordiceps sinensis within the same genus from various geographical areas were collected. After careful selection, purification and cleaning, the sexual portions of the heads were cut into small sections less than 5mm in length, then routinely sterilized for later usage.
(2) Preparations of culture media for test tube strains
The recipe for culture media preparation was as follows:

| | |
|---|---|
| Sugar | 1-5%, |
| Peptone | 0.1-1.0%, |
| Phosphates | 0.01-1%, |
| Sulphates | 0.01-1.0%, |
| Agrose | 0.5-5.0%, |
| Fluids from boiled wheat bran 5% | |
| Water | |

(3) The cultivation of hybrid strains
After the above step (2), the prepared culture media was placed in test tubes, inoculated with the fungus strains prepared in accordance with the above step (1) prepared, then incubated at 5-30^{¡£}C for 5-15 days. The storage number for the hybrid strains was CGMCCN00439, the classification name was Cordycops Sinensis, and the hybrids included both liquid and solid forms.
(4) Media preparations for fruitbody culture
The recipe was: foodstuff 100 units, dry chrysalis powder: 1-5 units, nutrient fluid: 0-90 units.
(5) Dispension of culture media
The media prepared in accordance with the above step (4) were dispensed into the culture containers at a thickness of 10-50 mm, closed tightly using air-controlling lids, thoroughly sterilized with routine method for late use.
(6) Hybrids inoculation for fruitbody culture
The media prepared in accordance with the above step (5) were inoculated with the hybrid strains prepared in accordance with the above step (3). The containers were placed in the chambers immediately after the inoculations. The cultures were maintained at 5-30°C under no-light conditions plus a relative humidity of 60-65 until the mycelium was fully spread on the media surface with complete penetration to the bottom. Then lighting was given at 200-400 lux for stimulating the differentiation of the primordium stroma. The temperature was maintained at 5-30°C and the relative humidity between 65-85. When rice-like primordium appeared on the media surface, the air conditioning units on the container lids were adjusted to provide a suitable amount of oxygen so as to gain the primordium stroma.
(7) Fruitbody culture
The primordium stroma produced in the above step (6) in the containers was transferred to the incubators or chambers of this invention. The temperature, humidity, light intensity and oxygen were adjusted to ensure normal growth of the stromas. The optimal temperature was between 5-30°C. The relative humidity was between 60-85 and the light intensity was between 200-400 Lux. The oxygen was increased gradually with the growth of the stroma, after about 20-90 days, the final mature fruitbodies emerged.
(8) Collection of fruitbodies

The mature fruitbodies were collected, while the immature ones were left for continued culture. The fruitbodies were dug out, with their nutriments exhausted and dried in the sun for use as ideal crude materials for the production of health-care products.

The sugar used in the above step (2) included frosted sugar, glucose, maltose or fructose. The foodstuff used in the above step (4) included rice, barley and mealies etc. and the nutrient fluid included sugar 0.1-5.0%, peptone 0.1-5.0%, ammonium citrate 0.05-1.0%, sulphate 0.01-0.5%, nutritious elements compounds 0-20%, vitamins 5-50 mg and water, PH 6-8. Among these components, the nutritious elements compounds were chosen from oxides, hydrates£¬acids, salines or organic compounds containing elements as N, P, K, Ca, Mg, S, B, Mo, Ce, La, Mn, Co, Cu, Zn, V, Ti or Fe.

The applications for Cordiceps sinensis include food tonics, immunization products or medicines.

The major advantages of this invention are:
1. That the cultivation devices and chambers invented here can establish suitable ecological environment for the growth and development of Cordiceps sinensis, thus greatly improve their yields and reduce the production costs for them due to maintenance of year-round growth and development of the fungus. The chambers are suitable for use in cultivation centers or factories; while the utensils can be introduced to individual families in some delicate sculpts and tiny forms thus provide nutriments for the customers with fresh fruitbodies at any time. In addition, the growing fruitbodies offer aesthetic appeal with their attractively changing colors and charming beauty.
2. That it created a fully new approach for the artificial culture of the fruitbodies of Cordiceps sinensis. Thus it not only generated an excellent quality of new fungus strains, but also a wholly new method for encarpiums culture with no restrictions in terms of time, place or any other factors. It not only improved yields and lowered costs, but also greatly enhanced the medicinal advantages. The active nucleosides were 17-fold higher than that in the wide species, and, surprisingly, the anti-tumor agent cordycepin was 345-fold higher than that in its wide peers according to analyses conducted by the Chinese Academy of Traditional Chinese Medicine

### Brief Illustrations to Figures

*Figure 1 sketch of the incubator body in section plane;*
*Figure 2 sketch of the cultivation container in section plane;*
*Figure 3 flowchart of the cultivation method of this invention.*

### The optimum methods of implementing this invention

### Sample 1

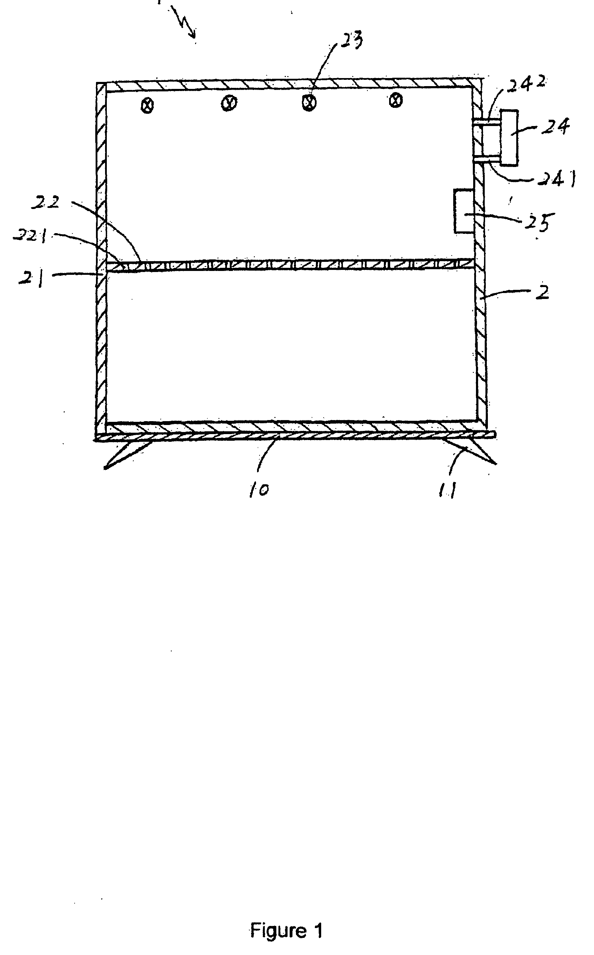

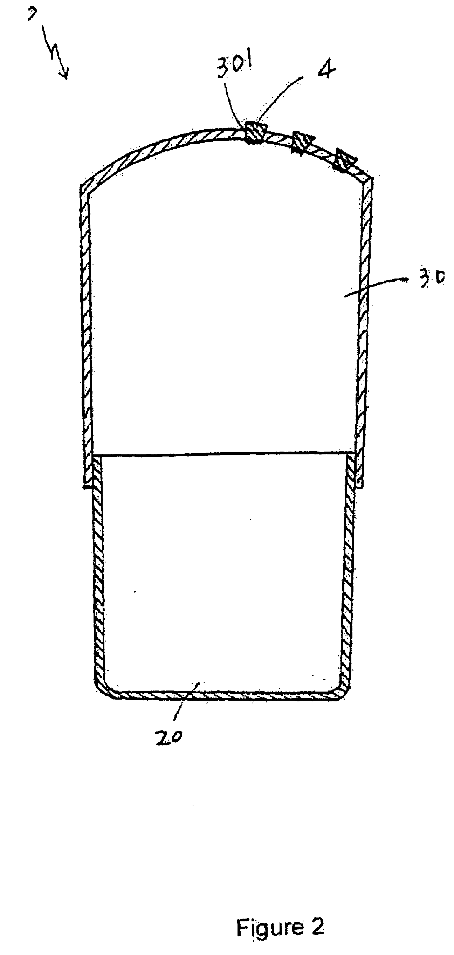

Please refer to figure 1 and 2; the cultivation devices include the incubator body 1 and the cultivation container 3. The body 1 includes the base 10 and the main body 2. There is/are door(s) 21 in the main body 2, with at least one interior layer 22 for holding the containers 3, the inlets 241 and outlets 242 for gases, with connections to the ventilation facilities 24, on the side faces. There are also air conditioning units 25 for controlling the temperature and the humidity together with the lighting facilities 23 within the main body 1. The container 3 includes a base 20 and a lid 30, comprising many holes 301 with elastic plugs 4 in them. There are many apertures 221 running through the interior layer(s) 22, or reticulation structures. The main bodies 2 of the incubators 1 are transparent or opaque; the lids 30 of the cultivation containers 3 are of transparent structures, while the bottoms 20 of the containers 3 are transparent or opaque. There are feet mountings 11 on the base 10, the incubator bodies 1 include bigger styles with multi-layers 22, suitable for uses in factories; the cultivation chambers with identical control units are suitable for uses in cultivation centers and factories. The incubator bodies 1 can also be made in delicate small styles for use with one or more containers 3 suitable for family use. They can still be made with unique artistic sculptures to enhance their appearance and for decorative purposes, while the cultivation containers can be made into sculptures of plants, animals or cartoons in combination with the attractive colors and shapes of the Cordyceps fruitbodies, for use as an attractive ornament as well as for ensuring adequate supplies of fresh fruitbodies for festive occasions.

The cultivation devices and chambers also included the mounting of computers and software systems to ensure optimum growth environments for the primordium fruitbodies via fully automatic control of the temperature, humidity and oxygen supplies so as to ensure a top-quality harvest of fruitbodies. Since the computer and software systems constitute pre-existing technology and are not the key points of this invention, they are not dealt with in any detail here.

### Sample 2

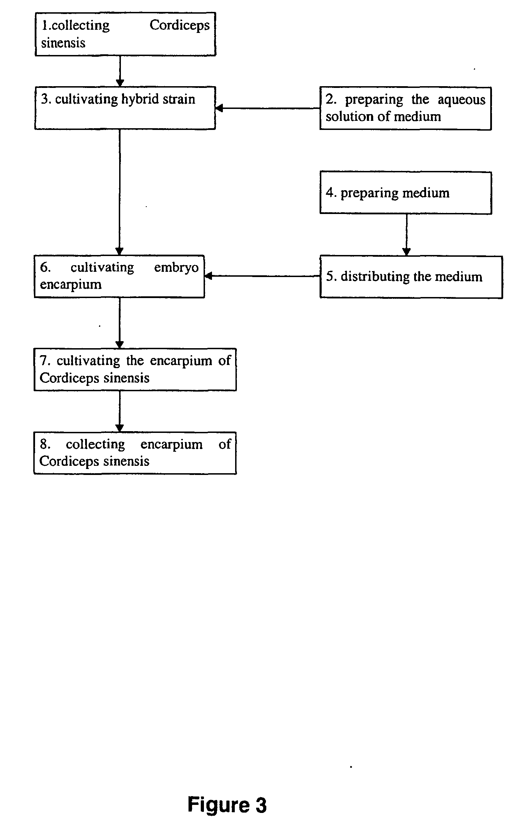

Figure 3, the cultivation method for Cordiceps sinensis in this invention includes the following steps:
(1) Various species of wide Cordiceps sinensis within the same genus were collected from different geographical areas. After careful selection£¬purification and cleaning, the sexual head portions were cut into small sections less than 5mm in length, and routinely sterilized for later use.
(2) Preparations of culture media for test tube strains
The recipe for culture media preparation was as follows:

| | |
|---|---|
| Sugar | 1-5%, |
| Peptone | 0.1-1.0%, |
| Phosphates | 0.01-1.0%, |
| Sulphates | 0.01-1.0%, |
| Agrose | 0.5-5.0%, |
| Fluids from boiled wheat bran 5% | |
| Water | |

(3) The cultivation of hybrid strains
The culture media prepared in accordance with the above step (2) was dispensed into test tubes and inoculated with the fungus strains prepared in accordance with the above step (1). It was then incubated at 5-30°C for 5-15 days to gain the hybrid fungus strains. The storage number for the hybrids was CGMCCN00439, the Classification Name was Cordycops Sinensis, and the strains included both liquid and solid forms.
(4) Preparations of culture media for fruitbody growth
The recipe was: foodstuff 100 units, dry chrysalis powder: 1-5 units, nutrient fluid: 0-90 units.
(5) Dispension of culture media into the container
The media prepared in accordance with the above step (4) were placed in the culture containers at a thickness of 10-50 mm, closed tightly with airing-controlled lids and thoroughly sterilized with routine methods for later use.
(6) Inoculation for culture of primordium fruitbodies
The hybrid strains prepared in accordance with the above step £^{··}3£©were placed in the media prepared in accordance with the above step (5) and put in the chambers immediately after the inoculations. The culture was maintained at 5-30°C under no-light conditions plus a relative humidity of 60-65 until the mycelium had fully spread on the media surface with complete penetration to the bottom. Then lighting was given at 200-400 lux to stimulate the differentiation of the primordium, the temperature was maintained at 5-30°C and the relative humidity was 65-85. When rice-like primordium appeared on the media surface, the air conditioning units on the container lids were adjusted to provide a suitable amount of oxygen so as to gain the primordium stroma.
(7) Fruitbody culture
The primordium stroma produced in accordance with the above step (6) was transferred to the incubators or chambers of this invention. The temperature, humidity, light intensity, and oxygen were adjusted to ensure the normal growth of the stromas. The optimum temperature was between 5-30°C, the relative humidity was 60-85 and the light intensity was between 200-400 Lux, the oxygen was increased gradually as the stroma grew. After some 20-90 days, the final matured fruitbodies were produced.
(8) Collection of fruitbodies
The mature fruitbodies were collected, while the immature ones were left for continued culture; the fruitbodies were dug out with nutriments exhausted and dried in the sun for use as ideal crude materials for making health care products. New batches of containers with primordium stromas were replaced so as to continue the culture throughout the entire year with greatly increased yields and lower costs. The containers with primordium stromas for family uses can be provided by culture centers or factories at any time.
The sugars referred to in the above step (2) included frosted sugar, glucose, maltose or fructose.
The hybrid strains referred to in the above step (3) included both liquid and solid forms.
The foodstuffs referred to in the above step (4) included rice, barley and mealies etc.
The chrysalis referred to in the above step (4) was powder from the ground silkworm chrysalis,
The nutrient fluid referred to in the above step (5) was a mixture of sugar 0.1-5.0%, peptone 0.1-5.0%, ammonium citrate 0.05-1.0%, sulphate 0.01-0.5%, nutritious elements compounds 2-20%, vitamins 5-50 mg, and water, PH 6-8.
The cultivation process referred to in the above step (7) in the incubators or chambers included supplying of adequate nutrient fluid to promote the healthy growth of the fruitbodies.
The nutritious elements compounds included oxides, hydrates, acids, salines or organics containing elements such as N, P, K, Ca, Mg, S, B, Mo, Ce, La, Mn, Co, Cu, Zn, V, Ti or Fe etc., ideally chosen from one or more of the following compounds such as HNO_{3.},H₃PO₄, KNO₃, (NH₄)₃PO₄, FeSO₄, Na₂B₄O₇, MnSO₄, MgSO₄, Ce₂(CO₃)₃,(NH₄)₆Mo₇O₂₄, ZnSO₄, La(OH)_{3,}, Ce(OH)_{3 ,} Mo₂O₃, ZnO or Co₃(PO₄)_{2.}

The fruitbodies from this invention possess higher nutrition and medicinal uses than those from the wide species. For details see annex setting out reports from the Chinese Academy of Traditional Chinese Medicine.

The uridine content was 3.1-fold higher than that in the wide species. Adenosine was 1.3, adenine 9.1 cordycepin 345.2, respectively; and uracil was present in traces. These figures fully indicated that the fruitbodies derived from this invention contained much higher nutritious and medicinal constituents than the wide species.

The applications of the fruitbodies derived from this invention include food tonics, immunization products or medicinal uses. For storage or transportation purposes, they can be dried and packaged with no impairment of their uses in tonic and remedies.

### Sample 3

Another implementation sample of this invention is as follows:
(1) Collection of fungus strains
Wide species of Cordiceps sinensis within the same genus from Tibet, Qinghai, Shichuan, Yunnan and Northeastern China were collected. The lusty fruitbodies were chosen for uses. After purification and cleaning, the sexual head portions were sectioned into small pieces of about 3 mm, disinfected with 0.1% corrosive sublimate HgCl₂₎ for 3 minutes, then washed thoroughly three times with sterile water and preserved for later use.
(2) Preparations of liquid media for test tube strains.
The recipe was as follows:

| | |
|---|---|
| White sugar | 3% |
| Peptone | 0.5% |
| KH₂PO₄ | 0.1% |
| MgSO₄ | 0.05% |
| Boiled liquid from white bran 5% | |
| Water balanced | |

(3) Cultivation of Hybrids
See Sample 2
(4) Media Preparations for Fruitbody Production
The recipe was as follows:

| | |
|---|---|
| Rice | 100 g |
| Dry chrysalis powder | 3g |
| Nutrient fluid | 60g |

The constituents of the Nutrient fluid were as follows: Add

| | |
|---|---|
| glucose | 1g |
| peptone | 0.5g |
| ammonium citrate | 0.1 g |
| MgSO₄ | 0.1g |
| Na₂B₄O₇ | 0.1g |
| ZnO | 0.2g |
| | |
| vitamin B | 10mg |

to100g of water; Final PH ∼7
(5) Media Dispension
The media prepared in accordance with the above step (4) were dispensed into the culture containers at a thickness of 30 mm, closed tightly with air-controlling lids. They were thoroughly sterilized routinely under normal or higher air pressure for later use.
In the following step (6) inoculations of fungus, step (7) cultivations in incubators or chambers and step (8), the fruitbodies harvested were all identical to those in sample 2, no repeats again.

### Sample 4

In this invention process, the recipe for preparations of the culture media according to step (4) was:

| | |
|---|---|
| Barley | 100g |
| Dry chrysalis powder | 2g |
| Nutrient fluid | 70g |

The compositions of the nutrient fluid were as follows:
Add

| | |
|---|---|
| maltose | 2g |
| peptone | 1g |
| ammonium citrate | 0.2g |
| MnSO₄ | 0.2g |
| ZnSO₄ | 0.1g |
| La(OH)_{3,} | 0.05g |
| | |
| vitamin compounds | 12 mg |

to 100g of water.

Others were the same as in sample 2, no repeats again.

### Sample 5

In this invention process, the recipe for preparations of the culture media according to step (4) was:

| | |
|---|---|
| soybean | 100g |
| dry chrysalis power | 2.5 g |
| nutrient fluid | 65 g |

The compositions of the nutrient fluid were as follows:
Add

| | |
|---|---|
| Fructose | 3g, |
| Peptone | 1.5g, |
| Ammonium Citrate | 0.15g |
| (NH₄)₃PO₄ | 1g |
| Ce₂(CO₃)₃ | 0.05g |
| Mo₂O₃ | 0.2g |
| Co₃(PO₄)_{2.} | 0.1g |
| Vitamins D | 15 mg |

to 100g of water. Otherwise the same as in sample 2, again no repetition.

### Sample 6

In this invention process, the recipe for preparations of the culture media according to step (4) was:

| | |
|---|---|
| mung bean | 100g |
| dry chrysalis powder | 3.1g |
| nutrient fluid | 75g |

the composition of the nutrient fluid was as follows:
Add

| | |
|---|---|
| white sugar | 3g, |
| peptone | 3 g, |
| ammonium Citrate | 0.5g |
| KNO₃ | 2g |
| H₃PO₄ | 3g |
| (NH₄)₆Mo₇O₂₄ | 1g |
| Ce(OH)₃ | 0.05g |
| vitamins D+E | 18g |

to 100g of water.

To sum up, the nutrient fluid is very important for the development and growth of the fruitbodies. Series of fruitbodies with various nutritious compositions for unique remedies uses can be produced using different elements by optionally adjusting the constituents of the media and their quotas. It has been demonstrated that the fruitbodies can be produced with no supplements of nutrient fluid in the culture media; however, it then takes longer for the fungus to mature and the resulting stroma is very delicate.

## Claims

1. A device for cultivation of Cordiceps sinensis, whose features cover incubator body and cultivation containers therein. The body of the incubator includes a base and a main body; in the main body, there is/are door(s) and at least one interior layer holding the containers. There are inlets and outlets for gases on the side for connection to ventilation facilities; air conditioning units for the control of temperature and humidity together with lighting facilities within the main body. In the cultivation container, there is a bottom and a lid, through which there may be many holes with elastic plugs in them.

2. According to rights claim 1, the cultivation device referred to covers the features of the interior layer(s) with apertures running through them, or it (they) include(s) reticulation structures.

3. According to rights claim 1, the cultivation device referred to covers features of their main bodies with transparent or opaque structures.

4. According to rights claim 1, the cultivation device referred to covers features of the cultivation containers with lids of transparent structures and bottoms of transparent or opaque structures

5. A method for the cultivation of Cordiceps sinensis, covering features as follows:
(1) Collections of fungus strains
Collect different species of wide Cordiceps sinensis within the same genus from various geographical areas. After careful selection and cleaning, cut the sexual heads portions into small sections less than 5mm in length, then routinely sterilize them for later uses.
(2) Preparations of culture media for test tube strains
The recipe for culture media preparation is as follows:
| | |
|---|---|
| Sugar | 1-5%, |
| Peptone | 0.1-1.0%, |
| Phosphates | 0.01-1%, |
| Sulphates | 0.01-1.0%, |
| Agrose | 0.5-5.0%, |
| Fluids from boiled wheat bran 5% | |
| Water | |
(3) The cultivation of hybrid strains
Dispense the culture media prepared in accordance with (2) above into test tubes, inoculate them with the fungus strains prepared in accordance with (1) above, then incubate the tubes for 5-15 days at 5-30°C. The storage number for the hybrid strains is CGMCCN00439, the classification name is Cordiceps sinensis. The hybrids include both liquid and solid forms.
(4) Preparations of culture media for fungus growth
The recipe is: rice 100 units, dry chrysalis power: 1-5 units, nutrient liquid: 0-90 units.
(5) Dispension of culture media into the container
Dispense the media prepared in accordance with the above step (4) into the culture containers to a thickness of 10-50 mm, close tightly with air-controlled lids. Thoroughly sterilize with routine methods for later use.
(6) Inoculation for culture of primordium fruitbodies
Inoculate the media prepared in accordance with (5) above with the hybrid strains prepared in accordance with (3) above. Place the containers in the chamber immediately after inoculations. Maintain the culture at 5-30°C under light-free conditions plus relative humidity of 60-65 until the mycelium has fully spread on the media surface with complete penetration to the bottom. Then provide lighting at 200-400 lux to stimulate the differentiation of the primordium. Maintain the temperature at 5-30°C and the relative humidity of 65-85. When rice-like primordium appears on the media surface, adjust the air conditioning units on the container lids to provide a suitable amount of oxygen so as to gain the primordium stroma.
(7) Fruitbody culture
Transfer the primordium stroma produced in accordance with the above step (6) together with their containers to the incubators or chambers of this invention, adjust the temperature, humidity, light intensity and oxygen to ensure the normal growth of the stromas. The optimum temperature is between 5-30°C, the relative humidity is between 60-85 and the light intensity is between 200-400 lux. The oxygen can be increased gradually with the growth of the stroma. After about 20-90 days, the fully matured fruitbodies can be harvested.
(8) Collection of fruitbodies
Collect the matured fruitbodies, leave the immature ones for continued culture. Dig out the primordium fruitbodies with their nutrients exhausted, dry them in the sun for use as ideal crude materials for making health care products.

6. According to rights claim 5, the method referred to covers features of sugars referred to in step (2) including white sugars, glucose, maltose or fructose.

7. According to rights claim 5, the method referred to covers features of the foodstuffs referred to in step (4) including rice, barley and mealies etc.

8. According to rights claim 5, the method referred to covers features of the nutrient fluid referred to in step (4), including sugar 0.1-5.0%, peptone 0.1-5.0%, ammonium citrate 0.05-1.0%, sulphate 0.01-0.5%, nutritious elements compounds 0-20%, vitamins 5-50mg ,and water. PH 6-8.

9. According to rights claim 8, the method referred to covers features of the nutritious elements compounds including oxides , hydrates£¬acids, salines or organic compounds containing elements as N, P, K, Ca, Mg, S, B, Mo, Ce , La, Mn, Co, Cu, Zn, V, Ti or Fe etc.

10. According to rights claim 8, the method referred to covers features of the nutritious elements compounds including those ideally chosen from one or more of the following compounds as HNO_{3.}, H₃PO₄, KNO₃, (NH₄)₃PO₄, FeSO₄, Na₂B₄O₇, MnSO₄, MgSO₄, Ce₂(CO₃)_{3,} (NH₄)₆Mo₇O₂₄, ZnSO₄, La(OH)₃, Ce(OH)_{3,} Mo₂O_{3,} ZnO or Co₃(PO₄)₂.

11. According to rights claim 5, the method referred to covers features of the components and their quotas of the fruitbody culture media: rice 100g, dry chrysalis powder 3g, nutrient fluid 60g. The constituents of the nutrient fluid are as follows: Glucose 1 unit, peptone 0.5 units, ammonium citrate 0.1 unit, MgSO₄ 0.1 unit, Na₂B₄O₇ 0.1 unit, ZnO 0.2 units, Vitamins B 0.01 unit, in 100 units of water

12. According to rights claim 5, the method referred to covers the features of step (4) for the components and their quotas of the fruitbodies culture media: barley 100 units, dry chrysalis powder 2 unit, nutrient fluid 70 units; the composition of the nutrient fluid is as follows: maltose 2 units, peptone 1 unit, ammonium citrate 0.2 unit MnSO₄ 0.2 units, ZnSO₄ 0.1 unit, La(OH)_{3,} 0.05 units, vitamin compounds 0.012 units in 100 units of water.

13. According to rights claim 5, the method referred to covers features of the components and their quotas of the fruitbody culture media: soybean 100 units, dry chrysalis powder 2.5 units, nutrient fluid 65 units; the composition of the nutrient liquid is as follows: fructose 3 units, peptone 1.5 units, ammonium citrate 0.2 unit, (NH₄)₃PO₄ 1 unit, Ce₂(CO₃)₃ 0.05 unit, Mo₂O₃ 0.2 units, Co₃(PO₄)_{2.} 0.1 unit, vitamin D 0.015 units in 100 units of water.

14. According to rights claim 5, the method referred to covers features of the components and their quotas of the fruitbody culture media: mung bean 100 units, dry chrysalis powder 3.1 units, nutrient fluid 75 units; the composition of the nutrient fluid is as follows: white sugar 3 units, peptone 3 units, ammonium citrate 0.5 units, KNO_{3.} 2 units, H₃PO₄ 3 units, (NH₄)₆Mo₇O₂₄ 1 unit, Ce(OH)₃ 0.05 units, Vitamins D+E 0.018 units in 100 units of water.

15. Applications of Cordiceps sinensis cover features in food tonics, immunization products or medical uses.
